# EUROPÄISCHE PATENTSCHRIFT

(11) **EP 1 825 256 B1**
(45) Veröffentlichungstag und Bekanntmachung des Hinweises auf die Patenterteilung: **10.02.2010**
(21) Anmeldenummer: 05818909.3
(22) Anmeldetag: 08.12.2005
(51) Int. Cl.: G01N 33/28

(54) **TESTMEDIUM ZUR SCHNELLANALYSE VON MOTOR\LEN IN VERBRENNUNGSMOTOREN**
TEST MEDIUM FOR THE RAPID ANALYSIS OF MOTOR OILS IN INTERNAL COMBUSTION ENGINES
MILIEU D'ESSAI POUR REALISER L'ANALYSE RAPIDE D'HUILES MOTEUR DANS DES MOTEURS A COMBUSTION INTERNE

(30) Priorität: 08.12.2004 DE 102004059020; 19.02.2005 DE 102005028025
(43) Veröffentlichungstag der Anmeldung: 29.08.2007
(73) Patentinhaber: Horstmeyer, Gert, 6681 Moersdorf (LU)
(72) Erfinder: Horstmeyer, Gert, 6681 Moersdorf (LU)
(74) Vertreter: Luderschmidt, Schüler & Partner
(86) Internationale Anmeldenummer: PCT/EP2005/013162
(87) Internationale Veröffentlichungsnummer: WO 2006/061222

(56) Entgegenhaltungen:
- WO-A-91/02579
- GB-A- 2 080 350
- US-A- 2 302 224
- US-A- 5 313 824
- PATENT ABSTRACTS OF JAPAN Bd. 016, Nr. 133 (C-0925), 6. April 1992 (1992-04-06) -& JP 03 296408 A (TOYO TOKUSHI KOGYO KK), 27. Dezember 1991 (1991-12-27) -& DATABASE WPI Section Ch, Week 199207 Derwent Publications Ltd., London, GB; Class J01, AN 1992-053043 XP002382767 & JP 03 296408 A (TOYO TOKUSHI KOGYO) 27. Dezember 1991 (1991-12-27)

## Beschreibung

Die vorliegende Erfindung betrifft ein Testmedium zur Schnellanalyse von Motorölen in Verbrennungsmotoren sowie ein Verfahren zur Schnellanalyse der aktuellen Zustände von Verbrennungsmotoren unter Verwendung des erfindungsgemäßen Testmediums.

Viele Gegenstände des täglichen Lebens, wie z. B. Autos, Motorräder, Bagger, Schiffe, Rasenmäher, werden heutzutage durch Verbrennungsmotoren angetrieben, wobei der Zustand des Verbrennungsmotors oftmals einen entscheidenden Einfluss auf die Brauchbarkeit und somit auf den Wert des Gegenstandes hat. Die genaue Beurteilung des Zustandes eines Verbrennungsmotors ist in der Regel, falls überhaupt, nur sehr schwer möglich, da sie meist die Zerlegung einzelner Aggregate oder den Ausbau des Verbrennungsmotors und seine Zerlegung in seine Einzelteile erfordert.

Alternativ ist es prinzipiell auch möglich, den Zustand des Verbrennungsmotors indirekt zu beurteilen. Hierzu kann beispielsweise eine Probe des im Verbrennungsmotor verwendeten Motoröls entnommen und in einem geeigneten Labor hinsichtlich seiner Zusammensetzung untersucht werden. Ein Vergleich des resultierenden Analyseergebnisses mit der Zusammensetzung des ursprünglich eingesetzten Motoröls liefert dann wertvolle Hinweise bzgl. des Zustandes des Verbrennungsmotors. Diese Vorgehensweise ist jedoch äußerst zeit- und kostenintensiv und daher in der Regel für die Praxis, beispielsweise beim Wiederverkauf des Gegenstandes, ungeeignet.
Die regelmäßige Überprüfung des Motorzustands lässt weiterhin ein rechtzeitiges Erkennen von Mängeln oder Fehlern am Motor zu und die Behebung dieser Mängel sind meist weniger aufwendig als ein neuer Motor.

Es besteht daher Bedarf nach einem einfachen, schnellen und kostengünstigen Verfahren zur Beurteilung des Zustandes eines Verbrennungsmotors.

Einen ersten Ansatz zur Lösung dieser Probleme bietet das Testpapier "Colutest^{®}" der Firma Stratex. Bei diesem Testpapier trägt man einen Tropfen des zu untersuchenden Motoröls auf ein Testblatt aus Cellulose und Baumwolle auf und lässt diesen in das Papier eindringen. Auf diese Weise erhält man eine chromatographische Auftrennung des Motoröls, mit deren Hilfe Verunreinigungen im Motoröl (Russ, Kühlmittel, Treibstoff) und Veränderungen des Motoröls (Oxidation des Motoröls) erkannt werden können. Nachteilig an diesem Test ist jedoch die extrem lange Testdauer von mehreren Stunden, meist von bis zu 12 oder gar 24 Stunden, die für die in der Praxis geforderte rasche Beurteilung des Zustandes z.B. von Gebrauchtwagen viel zu lang ist. Weiterhin führt der Test bei Dieselmotoren oft zu unscharfen und ungenauen Ergebnissen.

JP 03-296408 A offenbart ein Filtermedium für Bypass-Ölfilter, das eine Faser und einen Füllstoff enthält. Als Beispiel für die Faser werden Cellulosefasern, Lintasstoff, Rayon und Polyester namentlich genannt. Als Beispiel für den Füllstoff wird pulverisiertes Magnesiumhexasilicat erwähnt. Das Gewichtsverhältnis von Faser zu Füllstoff ist vorzugsweise 40/60 bis 90/10. Das Flächengewicht der Filtermedien beträgt in den Beispielen 250 g/m².

US 5 313 824 beschreibt ein Testkit zur Untersuchung von Motorölen, umfassend ein Chromatographiepapier. Jedoch werden weder der Baumwollpulp-Anteil im Testmedium noch das Flächengewicht des Testmediums beschrieben.

In Anbetracht dieses Standes der Technik war es daher Aufgabe der vorliegenden Erfindung, bessere und schnellere Möglichkeiten zur Beurteilung des Zustandes von Verbrennungsmotoren aufzuzeigen.

Gelöst werden diese sowie weitere Aufgaben, die zwar nicht wörtlich genannt werden, sich aber aus den hierin diskutierten Zusammenhängen wie selbstverständlich ableiten lassen oder sich aus diesen zwangsläufig ergeben, durch ein Testmedium mit allen Merkmalen des vorliegenden Anspruchs 1. Zweckmäßige Abwandlungen des erfindungsgemäßen Testmediums werden in den auf Anspruch 1 rückbezogenen Unteransprüchen beschrieben. Der unabhängige Verfahrensanspruch schützt eine besonders vorteilhafte Vorgehensweise zur Schnellanalyse von Motorölen in Verbrennungsmotoren. Ganz besonders geeignete Variationen des erfindungsgemäßen Verfahrens werden in den abhängigen Verfahrensansprüchen beschrieben.

Dadurch, dass man ein Testmedium zur Verfügung stellt, welches ein Flächengewicht von 50,0 bis 200,0 g/m² aufweist und, bezogen auf das Gesamtgewicht des Testmediums, 70,0 Gew.-% bis 98,0 Gew.-% Baumwollpulp, 0,0 Gew.-% bis 25,0 Gew. % Cellulose und 0,5 Gew.-% bis 30,0 Gew. % Kieselsäure und/oder mindestens eines Silikates umfasst, gelingt es auf nicht ohne weiteres vorhersehbare Weise, ein Testmedium zur Schnellanalyse von Motorölen in Verbrennungsmotoren zugänglich zu machen, welches eine vergleichsweise schnelle und einfache Beurteilung des Zustandes von Verbrennungsmotoren erlaubt.

Gleichzeitig ergeben sich durch die erfindungsgemäße Lösung noch die folgenden weiteren Vorteile:
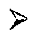 Das erfindungsgemäße Testmedium kann auf einfache Art und Weise, großtechnisch und kostengünstig hergestellt werden.
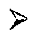 Das erfindungsgemäße Testmedium liefert in kurzer Zeit eindeutig interpretierbare Ergebnisse zum Zustand des Motoröls und gewährt somit einen schnellen, deutlichen Einblick in das Innere des Motors, wie es sonst nur mit Hilfe von aufwendigen und teuren Labortests möglich ist.
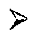 Das erfindungsgemäße Testmedium ermöglicht eine vergleichsweise genaue Analyse der Güte des zu untersuchenden Motoröls. Sowohl eine Alterung des Motoröls (Viskositätszunahme, Oxidation) als auch eine Verunreinigung des Motoröls, insbesondere durch Verbrennungsrückstände (Ruß), Treibstoff, Kühlflüssigkeit (Wasser, Glykol) kann auf einfache Art und Weise detektiert werden. Auf diese Weise lassen sich Fehler oder Mängel am Verbrennungsmotor erkennbar darstellen und eingrenzen.
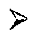 Durch die Analyse des Motoröls mit Hilfe des erfindungsgemäßen Testmediums erhält der Anwender wertvolle Hinweise und Informationen bezüglich eventuell bestehender Mängel.
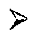 Durch die Analyse des Motoröls mit Hilfe des erfindungsgemäßen Testmediums kann der Anwender auf einfache Art und Weise überprüfen, ob oder wann ein aufgrund einer bestimmten Laufzeit oder Laufleistung üblicherweise vorgesehener Motorölwechsel notwendig ist.
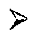 Das erfindungsgemäße Testmedium bietet eine einfache und gleichzeitig überaus effiziente Möglichkeit, den optimalen Wirkungsgrad und die optimale Verbrennung des Verbrennungsmotors zu prüfen. Nachteilige Auswirkungen auf die Umwelt wegen zu hohem Treibstoffverbrauch oder erhöhtem Ausstoß von Schadstoffen können auf diese Weise vermieden werden.
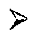 Das erfindungsgemäße Testmedium erlaubt die Schnellanalyse von allen Motoren, die mit einem Schmiermittel (Motoröl) betrieben werden und eignet sich insbesondere auch zur Schnellanalyse von Dieselmotoren.

Das erfindungsgemäße Testmedium weist ein Flächengewicht im Bereich von 50,0 bis 200,0 g/m², vorzugsweise im Bereich von 60,0 bis 140,0 g/m², insbesondere im Bereich von 70,0 bis 100,0 g/m², auf.

Weiterhin umfasst es, bezogen auf das Gesamtgewicht des Testmediums, 70,0 Gew,-% bis 98,0 Gew.-%, Baumwollpulp, 0,0 bis 25,0 Gew.-%, bevorzugt 0,0 bis 10,0 Gew.-%, besonders bevorzugt 0,0 bis 5,0 Gew.-%, insbesondere 0,0 Gew. % Cellulose, und 0,5 Gew.-% bis 30,0 Gew.-%, insbesondere 5,0 Gew.-% bis 30,0 Gew.-%, Kieselsäure und/oder mindestens eines Silikates.

Gemäß einer besonders bevorzugten Ausführungsform enthält das Testmedium, bezogen auf sein Gesamtgewicht, mindestens 0,1 Gew.-% Cellulose.

Die vorstehenden Bestandteile sind an sich bekannt. Unter Baumwollpulp versteht man die verspinnbaren und nicht-verspinnbaren Samenhaare des seit über 5000 Jahren in tropischen bis subtropischen Gegenden kultivierten, zu den Malvengewächsen zählenden, gelb blühenden Baumwollstrauches (Gossypium).

Cellulose bezeichnet das β-1,4-Polyacetal der Cellobiose, Eine erfindungsgemäß besonders bevorzugte Ausführungsform der Cellulose ist der aus Holz gewonnene Grundstoff zur Papierherstellung.

Die erfindungsgemäß einsetzbaren Silikate umfassen Salze und Ester, insbesondere Salze (sog. Kieselsäureester) der Ortholdeselsäure [Si(OH)₄] und deren Kondensationsprodukte, wie beispielsweise Nesosilikate (Inselsilikate), Inosilikate (Kettensilikate und Bandsilikate), Phyllosilikate (Blattsilikate, Schichtsilikate) und Tectosilikate (Gerüstsilikate). Erfindungsgemäß ganz besonders geeignete Silikate umfassen Aluminiumsilikat und Calciumsilikat. Ebenfalls gute Ergebnisse können unter Verwendung von Kaolin, Chinaclay und/oder Bullcaid (zuschlagstoffe auf Silikatbasis), bevorzugt in Mengen von 5,0 bis 30,0 Gew.-%, bezogen auf das Gesamtgewicht des Mediums erzielt werden. Für weitere Details wird auf die gängige Fachliteratur, insbesondere auf Römpp-Lexikon Chemie; Herausgeber: J. Falbe, M. Regitz; Stuttgart, New York; Thieme, 9. Auflage, Stichwort "Silicate" und die dort angegebenen Literaturstellen verwiesen,

Im Rahmen der vorliegenden Erfindung können die Silikate sowohl einzeln als auch in Mischung vorliegen.

Gemäß einer besonders bevorzugten Ausführungsform der vorliegenden Erfindung enthält das Testmedium mindestens eine vernetzte Kieselsäure,

Erfindungssemäß enthält das Medium, bezogen auf 0,031 m² Testmedium, vorzugsweise 0,1 g bis 1,86 g, insbesondere 0,1 g bis 1,5 g, Kieselsäure. Dabei umfasst das Papier gemäß einer ersten, ganz besonders bevorzugten Variante der vorliegenden Erfindung, bezogen auf 0,031 m² Testmedium, 0,1 g bis 0,6 g Kieselsäure. Gemäß einer weiteren, ganz besonders bevorzugten Variante der vorliegenden Erfindung umfasst das Papier, bezogen auf 0,031 m² Testmedium, 0,3 g bis 1,5 g Kieselsäure.

Der Silikatgehalt des Mediums, bezogen auf 0,031 m² Testmedium, liegt günstiger weise im Bereich von 0,1 g bis 1,5 g. Dabei enthält das Medium gemäß einer ersten, ganz besonders bevorzugten Ausführungsform der vorliegenden Erfindung, bezogen auf 0,031 m² Testmedium, 0,3 g bis 0,9 g Aluminiumsilikat. Gemäß einer zweiten, ganz besonders bevorzugten Ausführungsform der vorliegenden Erfindung umfasst das Medium, bezogen auf 0,031 m² Testmedium, 0,1 g bis 0,8 g Calciumsilikat oder ein anderes Silikat. Gemäß einer dritten, ganz besonders bevorzugten Ausführungsform der vorliegenden Erfindung umfasst das Medium, bezogen auf 0,031 m² Testmedium, 0,4 g bis 1,0 g Calciumsilikat.

Für die Zwecke der vorliegenden Erfindung ist es weiterhin bevorzugt, dass das Medium, bezogen auf 0,031 m² Testmedium, 0,01 g bis 0,1 g an sich bekannte Bindemittel und/oder Retentionsmittel enthält. Das Bindemittel soll dabei insbesondere zur Leimung des Testmediums, d. h. zur Verfestigung des Fasergefüges, zur Bindung von Füllstoffen und ggf. von Pigmenten, zur Erhöhung der Wasserfestigkeit und zur Verbesserung der Beschreib- und Bedruckbarkeit dienen. Das Retentionsmittel wird insbesondere zur Rückhaltung eventueller Fein- und Füllstoffe während der Herstellung zugegeben.

Erfindungsgemäß ganz besonders geeignete Bindemittel umfassen Stärke, Kasein, Proteine, Kunststoff-Dispersionen und Harzleime. Als Retentionsmittel haben sich erfindungsgemäß insbesondere Aluminiumsulfat sowie synthetische kationische Stoffe bewährt.

Im Rahmen der vorliegenden Erfindung enthält das Testmedium weiterhin, bezogen auf 0,031 m² Testmedium, 0,001 g bis 0,1 g, insbesondere 0,01 g, mindestens eines Entschäumers. Die in diesem Zusammenhang einsetzbaren Verbindungen sind aus dem Stand der Technik hinlänglich bekannt und unterliegen keinen besonderen Beschränkungen.

Die Herstellung der erfindungsgemäßen Trägermaterialen kann auf an sich bekannte Weise erfolgen. Sie umfasst vorzugsweise die folgenden Schritte:

### 1. Stoffaufbereitung

Die Zellstoffe werden vorzugsweise überwiegend in trockener Form angeliefert und im Pulper (Stoffauflöser) in Wasser zum pumpfähigen Faserbrei suspendiert. Die Faserstoff-Suspensionen durchlaufen dann vorzugsweise verschiedene Stationen der Reinigung, Mahlung (Fibrillieren und Kürzen der Fasern auf erforderliche Länge) und eventuell Klassieren (Trennen nach verschiedenen Faserlängen), bevor sie der "Stoffzentrale" zugleitet werden. In der Stoffzentrale werden die aufbereiteten Faserstoff-Suspensionen in einer "Mischbütte" vorzugsweise rezepturgerecht mit den - ggf. ebenfalls in wässrigflüssiger Form vorbereiteten Papierhilfsmitteln - zum sogenannten Ganzstoff zusammengemischt. Dieser besteht vorzugsweise aus 2,0 bis 6,0 Gew.-% Feststoff und 94,0 bis 98,0 Gew.-% Wasser und wird vor Aufgabe auf die Papiermaschine vorzugsweise weiter verdünnt.

### 2. Papiermaschine

In der Papiermaschine wird aus dem hoch verdünnten Ganzstoff kontinuierlich eine flächige Bahn erzeugt und das Wasser mittels mechanischer und thermischer Kräfte abgetrennt. Für die technischen Details wird auf die Fachliteratur, insbesondere auf Römpp-Lexikon Chemie; Herausgeber: J. Falbe, M. Regitz; Stuttgart, New York; Thieme, 9. Auflage, Stichwort "Papier" und die dort angegebenen Literaturstellen verwiesen.

### 3. Veredelung

Das aus der Papiermaschine kommende "maschinenglatte" Papier wird vorzugsweise mit Hilfe mindestens einer Streichanlage und/oder mindestens einem Kalander veredelt. Dabei wird in der Streichanlage das Papier einseitig oder beidseitig mit einer ein Bindemittel umfassendes Streichzusammensetzung versehen. Im Kalander wird der Papier-Oberfläche Glätte und Glanz verliehen. Für weitere Details wird wiederum auf die Fachliteratur, insbesondere auf Römpp-Lexikon Chemie; Herausgeber: J. Falbe, M. Regitz; Stuttgart, New York; Thieme, 9. Auflage, Stichwort "Papier" und die dort angegebenen Literaturstellen verwiesen.

### 4. Ausrüstung

Letzter Prozessschritt der Herstellung ist die als Ausrüstung bezeichnete Zerteilung der von der Papiermaschine oder von der Veredelung kommenden Großrollen in die benötigten Formate. Weitere Details können der Fachliteratur, insbesondere aus Römpp-Lexikon Chemie; Herausgeber: J. Falbe, M. Regitz; Stuttgart, New York; Thieme, 9. Auflage, Stichwort "Papier" und den dort angegebenen Literaturstellen entnommen werden.

Im Rahmen einer ersten ganz besonders bevorzugten Ausführungsform der vorliegenden Erfindung erfolgt die Herstellung des erfindungsgemäßen Testmediums, indem man die Baumwollpulp sowie ggf. vorhandene Cellulose und die Kieselsäure und/oder das mindestens eine Silikat in den jeweils gewünschten Mengen gemäß dem vorstehenden Verfahren zu einem Ganzstoff vermischt und zu einem Testpapier verarbeitet.

Gemäß einer zweiten ganz besonders bevorzugten Ausführungsform der vorliegenden Erfindung erfolgt die Herstellung des erfindungsgemäßen Testmediums, indem man gemäß dem vorstehend beschriebenen Verfahren ein "maschinenglattes Papier" herstellt, welches die erforderlichen Anteile von Baumwollpulp und ggf. Cellulose enthält, und dieses in einer speziellen Vorrichtung mit den benötigten Mengen Kieselsäure und/oder des mindestens einen Silikates imprägniert.

Gemäß einer dritten ganz besonders bevorzugten Ausführungsform der vorliegenden Erfindung erfolgt die Herstellung des erfindungsgemäßen Testmediums, indem man gemäß dem vorstehend beschriebenen Verfahren ein "maschinenglattes Papier" herstellt, welches die erforderlichen Anteile von Baumwolle und ggf. Cellulose enthält, und dieses mit einer Streichzusammensetzung bestreicht, die die benötigten Mengen der Kieselsäure und/oder des mindestens einen Silikates enthält.

Die Anwendung des erfindungsgemäßen Testmediums ist dem Fachmann aufgrund der vorstehend beschriebenen Zusammenhänge unmittelbar offensichtlich. Vorzugsweise wird es zur Schnellanalyse des Motoröles in einem Verbrennungsmotor verwendet, wobei man einen Tropfen des zu untersuchenden Motoröls, vorzugsweise punktförmig, auf ein Testmedium gemäß mindestens einem der vorangehenden Ansprüche aufträgt und in das Medium eindringen lässt.

Die vorliegende Erfindung gibt bereits durch die Eintrockengeschwindigkeit Auskunft über den Zustand des Motoröls, d. h. je schneller das Motoröl eingezogen ist, umso besser ist der Zustand des Motoröls (gute Viskosität) und je länger es benötigt, um in das Testmedium einzudringen, umso schlechter ist die Schmierkraft (hohe Viskosität, also schlechter Zustand des Motoröls). Dementsprechend wird beim erfindungsgemäßen Verfahren vorzugsweise die Eindringzeit des Motoröls in das Testmedium gemessen.

Durch die Auswertung der resultierenden Trennung auf dem Testmedium erhält der Fachmann wertvolle Hinweise, insbesondere hinsichtlich:
■ der Viskosität des Motoröls, die vorzugsweise zumindest qualitativ bestimmt wird;
■ des Oxidationsgrads des Motoröls, der vorzugsweise zumindest qualitativ bestimmt wird;
■ des Russanteils im Motoröl, der vorzugsweise zumindest qualitativ bestimmt wird;
■ des Wassergehalts im Motoröl, der vorzugsweise zumindest qualitativ bestimmt wird;
■ des Glykolgehalts im Motoröl, der vorzugsweise zumindest qualitativ bestimmt wird;
■ einer ggf. vorliegende Verdünnung des Motoröls mit Kraftstoff, die vorzugsweise zumindest qualitativ bestimmt wird;
■ des Gehalts an Staub im Motoröl, der vorzugsweise zumindest qualitativ bestimmt wird.

Zu diesem Zweck wird die resultierende Trennung der jeweiligen Öl-Bestandteile vorzugsweise mit mindestens einem Referenzbild verglichen. Die Referenzbilder zeigen dabei zweckmäßigerweise die einzelnen Zustandsstufen des Motoröls und ermöglichen dem Fachmann und dem Laien gleichermaßen, die richtigen Schlüsse aus dem durchgeführten Test zu ziehen.

Beispielsweise können Verbrennungsrückstände (Ruß im Motoröl) auf eine schlechte Einstellung des Vergasers, eine mangelhafte Einspritzung, eine schlechte Vergasung, eine falsch eingestellte Einspritzpumpe, eine schlechte Luftzufuhr und/oder eine falsche Fahrweise hinweisen, die zu einer Verschmutzung des Motors und des Motoröls sowie der Ölkanäle, zu einer Verminderung der Arbeitspielräume, zu einer Erhitzung des oberen Motorteils und/oder zu einem erhöhten Verschleiß der Reibeflächen führen können.

Ein schlechter Zustand des Motoröls kann beispielsweise auf ein zu altes Motoröl, auf die Gegenwart von Kühlwasser im Motoröl und/oder auf die Bildung von Metallteilchen zurückzuführen sein und eine Verminderung der Viskosität des Motoröls, einen Säureangriff auf Metallteile im Verbrennungsmotor (Blei, Kupfer, Motorwanne und untere Teile) und/oder einen höheren Verschleiß der Kolbenringe und der Zylinder bedingen.

Treibstoffbestandteile im Motoröl können beispielsweise durch eine mangelhafte Einspritzung, durch eine schlechte Einstellung des Vergasers, durch einen Defekt der Einspritzdüsenöffnung, durch eine schlechte Zündeinstellung, durch verschmutzte Ansaugluft, durch zu hochtouriges Fahren mit kaltem Motor und/oder durch Kurzstreckenfahrten bedingt sein, die zu einem erhöhten Treibstoffverbrauch, zu einer Beeinträchtigung der Steuerkette, zu einer Verdünnung des Motoröls und dadurch zu der Gefahr der Überhitzung des oberen Motorbereichs, zu einer Verminderung der Schmierfähigkeit des Motoröls und/oder zu einem erhöhten Verschließ der Reibflächen (Kolben, Zylinder) führen können.

Wasser und/oder Kühlflüssigkeit im Motoröl kann beispielsweise von einer undichten Zylinderkopfdichtung, von porösen Dichtungen (insbesondere die Ölkühlerdichtung), von einem undichten Wasserkühlsystem, von verschmutzter Ansaugluft und/oder von Kondenswasser, beispielsweise infolge vieler Kurzstreckenfahrten, herrühren. Mögliche Folgen sind eine Oxidation des Motoröls, eine Verdünnung des Motoröls, eine Erhöhung der Öltemperatur und des Druckes sowie ein erhöhter Motorverschleiß.

In allen vorstehend beschriebenen Fällen empfehlen sich eine gründliche Überprüfung der genannten möglichen Ursachen und die Durchführung eines Motorölswechsels. Der Test sollte nach einer Laufleistung von ca. 100 bis 500 km wiederholt werden, um sicherzustellen, dass die Mängel vollständig behoben wurden.

Günstigerweise wird bei der Schnellanalyse des Motoröls so vorgegangen, dass man die resultierende Trennung der Öl-Bestandteile 1 Sekunde bis 180 Minuten, vorzugsweise 1 Minute bis 120 Minuten, insbesondere 2 Minuten bis 60 Minuten, nach dem Auftragen nur eines Tropfens Motoröl auswertet. Die erhaltenen Ergebnisse werden vorzugsweise dokumentiert, damit man einen Überblick über die Entwicklung des Verbrennungsmotors erhält und auf eventuelle Abweichungen sofort reagieren kann.

Die mit den Motoröl verunreinigten Testmedien können auf einfache Art und Weise gesammelt und umweltgerecht entsorgt werden.

Nachfolgend werden die Ergebnisse von Versuchen und Vergleichsversuchen beschrieben, ohne dass hierdurch eine Beschränkung des Erfindungsgedankens erfolgen soll.

Als Trägermaterial wurde für die bisher am erfolgreichsten Ergebnisse 100 % Baumwollpulp mit Flächengewichten von 50 g/m² bis 200 g/m² und insbesondere mit Flächengewichten von 60 g/m² bis 140 g/qm² verwendet.

Die Katalysatoren, Beschleuniger und Reaktionsmittel sind in den besten Ergebnissen (Gewichte bezogen auf ein Testblatt mit einem Durchmesser von 200 mm):
○ 0,3 g bis 0,9 g Aluminiumsilikat, 0,1 g bis 0,6 g gefällte Kieselsäure, 0,01 g bis 0,1 g Bindemittel und Retentionsmittel, 0,01 g Entschäumer.
○ 0,3 g bis 1,5 g gefällte Kieselsäuren und 0,1 g bis 0,8 g Calciumsilikat, 0,01 g bis 0,1 g Bindemittel und Retentionsmittel, 0,01 g Entschäumer. Einige Kieselsäuren wurden wegen der hohen Hydrophobität speziell vernetzt.
○ 0,4 g bis 1,0 g Calciumsilikat, 0,01 g bis 0,1 g Retentions- und Bindemittel und Entschäumer.

Ebenfalls gute Ergebnisse wurden mit Baumwoll-Papieren, 140 g/m², mit der Zugabe von jeweils 5 - 30 Gew.-% Kaolin, Chinaclay und/oder Bullcaid erzielt, wobei die Eindringzeiten gut, die Reifeprozesse jedoch vergleichsweise langsam waren.

Die Beurteilung der erzielten Ergebnisse fand in Bezug und der Möglichkeiten der Testblätter von Anbietern des Standes der Technik statt. Hierbei handelte es sich um Papiere, die aus Cellulose und Baumwolle bestehen und zwar im Verhältnis 90:10 oder 80:20. Bei diesen Medien wurden die Endergebnisse im Regelfall erst nach 10 bis 24 Stunden erhalten. Bei älteren, verbrauchten Motorölen dauerte die Eindringzeit der Öle extrem lange, bis zu mehreren Stunden. Auch waren die Endergebnisse mit Motorölen aus Dieselfahrzeugen zu unscharf und an den Übergängen nicht deutlich genug.

Handelsübliche Laborfilterpapiere können mit dem erfindungsgemäßen Testmedium gut verglichen werden; sie weisen ähnliche Eigenschaften wie die beschriebenen Testblätter des Standes der Technik auf. Hierbei wurden zahlreiche Papiersorten und Qualitäten u. a. von Schleicher & Schüll und von der Macherei Nagel getestet. Auch hier dauerten die Eindring- und Reifezeiten vor allem bei alten Ölen sehr lange und die Dieselmotoröle waren ebenfalls zu undeutlich.

Von den Endergebnissen überzeugend erwiesen sich die Versuche mit DC- Plättchen und DC-Papieren (Dünnschicht-Chromatographie) auf Basis von Aluminiumsilikaten und Kieselsäuren und Kieselgel. Aber auch hier erwies sich die Trockenzeit als unakzeptabel, da insbesondere ältere und verbrauchte Öle selbst nach Tagen nicht trockneten.

Inkjet-Papiere zeigten bei der Trennung der Ölbestandteile ebenfalls im Endergebnis gute Resultate, aber auch diese waren, wie die DC-Alufolien und viele dichte Papiere bei der Trocknung und Reifezeit wesentlich zu langsam; die Zeiten lagen zwischen etwa 1 Stunde und mehreren Tagen.

Die Beurteilungskriterien der Versuche (in Protokollen von 0 = schlecht bis 10 = sehr gut) waren wie folgt:
■ Eindringzeit des Motoröls (nicht die endgültige Trocknung des Motoröls!)
■ Trennung der Bestandteile (Ruß)
■ Erkennung der Viskosität
■ Bildung von hellen Ringen (Treibstoff im Öl)
■ Zackenbildung am Rand (Wasser im Öl) und Bildung der Korona (Glykol)
■ Schlierenbildung im Endergebnis (nach endgültiger Trocknung)

Diese Zeiten bewegten sich bei den erfindungsgemäßen Produkten in folgenden Bereichen:

| | | |
|---|---|---|
| ■ | Eindringzeit des Motoröls | 2 bis 30 Minuten |
| ■ | Trennung der Bestandteile (Ruß) | 2 bis 60 Minuten |
| ■ | Erkennung der Viskosität | 5 bis 30 Minuten |
| ■ | Bildung von hellen Ringen (Treibstoff im Öl) | 2 bis 30 Minuten |
| ■ | Zackenbildung am Rand (Wasser im Öl) | 1 bis 60 Minuten |
| ■ | Bildung der Korona (Glykol) | 30 bis 180 Minuten |

Die Schlierenbildung erfolgt je nach Zustand des jeweiligen Öls nach einigen Stunden, da für diese Ergebnisse die endgültige Trocknung des Öls erfolgt sein muss.

## Patentansprüche

1. Testmedium zur Schnellanalyse von Motorölen in Verbrennungsmotoren,
aufweisend ein Flächengewicht von 50,0 bis 200,0 g/m²,
umfassend, bezogen auf das Gesamtgewicht des Testmediums,
70,0 Gew.-% bis 98,0 Gew.-% Baumwollpulp,
0,0 Gew.-% bis 25,0 Gew.-% Cellulose und
0,5 Gew.-% bis 30,0 Gew. % Kieselsäure und/oder mindestens eines Silikates.

2. Testmedium nach Anspruch 1, **dadurch gekennzeichnet, dass** das Testmedium, bezogen auf 0,031 m² Testmedium, 0,1 g bis 1,86 g Kieselsäure enthält.

3. Testmedium nach Anspruch 1 oder 2, **dadurch gekennzeichnet, dass** das Testmedium, bezogen auf 0,031 m² Testmedium, 0,1 g bis 1,5 g mindestens eines Silikates enthält.

4. Testmedium nach mindestens einem der vorangehenden Ansprüche, **dadurch gekennzeichnet, dass** das Testmedium Aluminiumsilikat und/oder Calciumsilikat enthält.

5. Testmedium nach mindestens einem der vorangehenden Ansprüche, **dadurch gekennzeichnet, dass** das Testmedium, bezogen auf 0,031 m² Testmedium, 0,01 bis 0,1 g Bindemittel und/oder Retentionsmittel enthält.

6. Testmedium nach mindestens einem der vorangehenden Ansprüche, **dadurch gekennzeichnet, dass** das Testmedium, bezogen auf 0,031 m² Testmedium, 0,001 g bis 0,1 g mindestens eines Entschäumers enthält.

7. Verfahren zur Schnellanalyse von Motorölen in Verbrennungsmotoren, bei welchem man einen Tropfen eines zu untersuchenden Motoröls auf ein Testmedium gemäß mindestens einem der vorangehenden Ansprüche aufträgt und in das Testmedium eindringen lasst.

8. Verfahren nach Anspruch 7, **dadurch gekennzeichnet, dass** man das Motoröl punktförmig auf das Testmedium aufträgt.

9. Verfahren nach Anspruch 7 oder 8, **dadurch gekennzeichnet, dass** man die Eindringzeit des Motoröls in das Testmedium misst.

10. Verfahren nach mindestens einem der Ansprüche 7 bis 9, **dadurch gekennzeichnet, dass** man die resultierende Trennung der Öl-Bestandteile auswertet.

11. Verfahren nach Anspruch 10, **dadurch gekennzeichnet, dass** man die resultierende Trennung der Öl-Bestandteile mit mindestens einem Referenzbild vergleicht.

12. Verfahren nach Anspruch 10 oder 11, **dadurch gekennzeichnet, dass** man die resultierende Trennung der Öl-Sestandteile 1 Sekunde bis 180 Minuten nach dem Auftragen des Motoröls auswertet.

13. Verfahren nach mindestens einem der Ansprüche 7 bis 12, **dadurch gekennzeichnet, dass** man die Viskosität des Motoröls zumindest qualitativ bestimmt.

14. Verfahren nach mindestens einem der Ansprüche 7 bis 13, **dadurch gekennzeichnet, dass** man den Oxidationsgrad des Motoröls zumindest qualitativ bestimmt.

15. Verfahren nach mindestens einem der Ansprüche 7 bis 14, **dadurch gekennzeichnet, dass** man den Russanteil im Motoröl zumindest qualitativ bestimmt.

16. Verfahren nach mindestens einem der Ansprüche 7 bis 15, **dadurch gekennzeichnet, dass** man den Wassergehalt im Motoröl zumindest qualitativ bestimmt.

17. Verfahren nach mindestens einem der Ansprüche 7 bis 16, **dadurch gekennzeichnet, dass** man den Glykolgehalt im Motoröl zumindest qualitativ bestimmt.

18. Verfahren nach mindestens einem der Ansprüche 7 bis 17, **dadurch gekennzeichnet, dass** man eine ggf. vorliegende Verdünnung des Motoröls mit Kraftstoff zumindest qualitativ bestimmt.

19. Verfahren nach mindestens einem der Ansprüche 7 bis 18, **dadurch gekennzeichnet, dass** man den Gehalt an Staub im Motoröl zumindest qualitativ bestimmt.

20. Verfahren zur Schnellanalyse des aktuellen Zustands eines Verbrennungsmotors, bei welchen man ein Testmedium gemäß mindestens einem der Ansprüche 1 bis 6 einsetzt.

## Claims

1. A test medium for the rapid analysis of engine oils in internal combustion engines,
having a weight per unit area of 50.0 to 200.0 g/m²,
comprising, based on the total weight of the test medium,
70,0% by weight to 98.0% by weight of cotton pulp,
0.0% by weight to 25.0% by weight of cellulose and
0.5% by weight to 30.0% by weight of silicic acid and/or at least one silicate.

2. The test medium according to claim 1, **characterized in that** the test medium contains, based on 0.031 m² of test medium, 0.1 g to 1,86 g of silicic acid.

3. The test medium according to claim 1 or 2, **characterized in that** the test medium contains, based on 0.031 m² of test medium, 0.1 g to 1.5 g of at least one silicate.

4. The test medium according to at least one of the preceding claims, **characterized in that** the test medium contains aluminium silicate and/or calcium silicate.

5. The test medium according to at least one of the preceding claims, **characterized in that** the test medium contains, based on 0.031 m² of test medium, 0.01 to 0.1 g of binder and/or retention agent.

6. The test medium according to at least one of the preceding claims, **characterized in that** the test medium contains, based on 0.031 m² of test medium, 0.001 g to 0.1 g of at least one defoamer.

7. A method for the rapid analysis of engine oils in internal combustion engines, in which a drop of an engine oil to be analysed is applied to a test medium according to at least one of the preceding claims and is allowed to penetrate into the test medium.

8. The method according to claim 7, **characterized in that** the engine oil is applied to the test medium in spot form.

9. The method according to claim 7 or 8, **characterized in that** the penetration time taken for the engine oil to penetrate into the test medium is measured.

10. The method according to at least one of claims 7 to 9, **characterized in that** the resulting separation of the oil components is evaluated.

11. The method according to claim 10, **characterized in that** the resulting separation of the oil components is compared with at least one reference image.

12. The method according to claim 10 or 11, **characterized in that** the resulting separation of the oil components is evaluated 1 second to 180 minutes after the application of the engine oil.

13. The method according to at least one of claims 7 to 12, **characterized in that** the viscosity of the engine oil is determined at least qualitatively.

14. The method according to at least one of claims 7 to 13, **characterized in that** the degree of oxidation of the engine oil is determined at least qualitatively.

15. The method according to at least one of claims 7 to 14, **characterized in that** the carbon black content in the engine oil is determined at least qualitatively.

16. The method according to at least one of claims 7 to 15, **characterized in that** the water content in the engine oil is determined at least qualitatively.

17. The method according to at least one of claims 7 to 16, **characterized in that** the glycol content in the engine oil is determined at least qualitatively.

18. The method according to at least one of claims 7 to 17, **characterized in that** any dilution of the engine oil with fuel is determined at least qualitatively.

19. The method according to at least one of claims 7 to 18, **characterized in that** the dust content in the engine oil is determined at least qualitatively.

20. A method for the rapid analysis of the actuell engine status, in which a test medium according to the claims 1 to 16 is applied.

## Revendications

1. Milieu d'essai pour réaliser l'analyse rapide d'huiles moteur dans des moteurs à combustion interne, ayant un poids de surface entre 50,0 et 200,0 g/m², et comprenant par rapport au poids total du milieu d'essai :
70,0 % de poids jusqu'à 98,0 % de poids de pulpe de coton
0,0 % de poids jusqu'à 25,0 % de poids de cellulose, et
0,5 % de poids jusqu'à 30,00 % de poids de silice et/ou d'au moins un silicate.

2. Milieu d'essai selon revendication 1, **caractérisé en ce que** le milieu d'essai, par rapport au 0,031 m² du milieu d'essai contient entre 0,1 g et 1,86 g de silice.

3. Milieu d'essai selon revendications 1 ou 2, **caractérisé en ce que** le milieu d'essai, par rapport au 0,031 m² du milieu d'essai contient entre 0,1 g et 1,5 g d'au moins un silicate.

4. Milieu d'essai selon au moins une des revendications précédentes, **caractérisé en ce que** le milieu d'essai contient de silicate d'aluminium et/ou de silicate de calcium.

5. Milieu d'essai selon au moins une de revendications précédentes, **caractérisé en ce que** le milieu d'essai, par rapport au 0,031 m² du milieu d'essai, contient entre 0,01 et 0,1 g de liant et/ou d'agent de rétention.

6. Milieu d'essai selon au moins une des revendications précédentes, **caractérisé en ce que** le milieu d'essai, par rapport au 0,031 m² du milieu d'essai, contient entre 0,001 et 0,1 g d'au moins un dé-mousseur.

7. Milieu d'essai pour réaliser l'analyse rapide d'huiles moteur dans des moteurs à combustion interne, dans le quel on applique une goutte d'huile moteur à analyser sur un milieu d'essai selon au moins une des revendications précédentes, en la laissant pénétrer dans le milieu d'essai.

8. Procédé selon revendication 7, **caractérisé en ce qu'**on applique l'huile moteur sur le milieu d'essai sous forme de points.

9. Procédé selon revendication 7 ou 8, **caractérisé en ce qu'**on mesure le temps de pénétration d'huile moteur dans le milieu d'essai.

10. Procédé selon une des revendications 7 à 9, **caractérisé en ce qu'**on évalue la séparation résultante des composants d'huile.

11. Procédé selon revendication 10, **caractérisé en ce qu'**on compare la séparation résultante des composants d'huile avec au moins une image de référence.

12. Procédé selon une des revendications 10 ou 11, **caractérisé en ce qu'**on évalue la séparation résultante des composants d'huile entre 1 second et 180 minutes après l'application d'huile moteur.

13. Procédé selon au moins une des revendications 7 à 12, **caractérisé en ce qu'**on détermine la viscosité d'huile moteur au moins sur un niveau qualitatif.

14. Procédé selon au moins une des revendications 7 à 13, **caractérisé en ce qu'**on détermine le niveau d'oxydation d'huile moteur au moins sur le niveau qualitatif.

15. Procédé selon au moins une des revendications 7 à 14, **caractérisé en ce qu'**on détermine la quote-part de suie d'huile moteur au moins sur le niveau qualitatif.

16. Procédé selon au moins une des revendications 7 à 15, **caractérisé en ce qu'**on détermine la quote-part d'eau d'huile moteur au moins sur le niveau qualitatif.

17. Procédé selon au moins une des revendications 7 à 16, **caractérisé en ce qu'**on détermine le contenu de glycol d'huile moteur au moins sur le niveau qualitatif.

18. Procédé selon au moins une des revendications 7 à 17, **caractérisé en ce qu'**on détermine une éventuelle dilution d'huile moteur avec le combustible au moins sur le niveau qualitatif.

19. Procédé selon au moins une des revendications 7 à 18, **caractérisé en ce qu'**on détermine le contenu de poussière dans l'huile moteur au moins sur le niveau qualitatif.

20. Procédé pour l'analyse rapide des conditions actuelles d'un moteur à combustion, dans le quel on utilise un milieu d'essai selon au moins une des revendications 1 à 6.
